# EUROPEAN PATENT SPECIFICATION

(11) **EP 4 057 958 B1**
(45) Date of publication and mention of the grant of the patent: **16.07.2025**
(21) Application number: 20804534.4
(22) Date of filing: 11.11.2020
(51) Int. Cl.: A61F 7/12, A61F 7/00

(54) **DEVICE FOR CEREBRAL TEMPERATURE CONTROL**
VORRICHTUNG ZUR HIRNTEMPERATURSTEUERUNG
DISPOSITIF DE RÉGULATION DE LA TEMPÉRATURE CÉRÉBRALE

(30) Priority: 13.11.2019 SE 1951308
(43) Date of publication of application: 21.09.2022
(73) Proprietor: Teqcool AB, 223 59 Lund (SE)
(72) Inventor: ALLERS, Mats, 226 50 LUND (SE)
(74) Representative: KIPA AB
(86) International application number: PCT/EP2020/081774
(87) International publication number: WO 2021/094381

(56) References cited:
- WO-A1-2005/087156
- WO-A1-2013/142365
- JP-A- 2000 325 388
- US-A1- 2010 324 483

## Description

### Field of invention

The invention relates to a device and method for indirect temperature regulation of the brain. The device and method are especially adapted for humans.

### Technical Background

In pathological conditions, the body temperature or the temperature of body parts of a human being influences the healing process and the risk of permanent damage. Cancer cells, for example, are heat sensitive and a local heating of the blood flow around a cancer tumour may for some types of cancer constitute a treatment resulting in restrained tumour growth or in some cases even in a shrinking of the tumour. In other cases, cooling of a body part may be important to reduce adverse secondary symptoms of the pathological condition, this is primarily intended for the treatment of brain ischemia. In the case of a stroke, the blood flow to the brain is reduced (ischemia) due to a haemorrhage or the clogging of a blood vessel. This condition will cause permanent functional deficits unless treatment to restore blood flow and protect nerve cells is initiated at an early stage, which will reduce the loss of bodily functions, such as paralysis. It is well known that cooling the brain effectively blocks the development of cellular damage after an episode of ischemia. Whole body cooling of the patient suffering transient circulatory arrest to the brain results in a reduction of the symptoms of neurological deficit. However, there are certain problems associated with whole body cooling. One is that the cooling is not fast enough to effectively use its protective potential. Another problem is that whole body cooling must be carried out under close control of physiological parameters or under anesthesia. Yet another problem is that there is a risk of cardiovascular complications. In the case of a circulatory arrest, the brain can suffer permanent damage if the arrest exceeds a time period of about 5-15 minutes. However, if the temperature of the brain is lowered before, during or after the arrest, the brain damage is diminished. In the case of brain trauma, the brain suffers from open or closed head concussion. Hypothermia has been shown to diminish traumatic brain injury in such cases.

Thus, there is a need for improvements in the field of brain temperature regulation.

WO 2005/087156 A1 discloses a system for cerebral temperature control of a human being, comprising a double lumen catheter to be introduced through a nostril of a human being and to be placed with its tip at the level of the back of the tongue. The double lumen catheter means comprises a first lumen and a second lumen being in fluid communication by means of a set of end openings. The second lumen is configured as an expandable balloon. A temperature regulator is connected to the reservoir comprising the cooling fluid for regulating the temperature of the fluid. A pump circulates the fluid. Alternatively, the fluid is fed by gravity.

### Summary of the invention

It is an object of the invention to provide an improved and efficient device and method for regulating the temperature of the brain via a nasal cavity without substantially changing the temperature of the rest of the body.

The invention is defined in the appended independent claim 1, further embodiments are described in the dependent claims.

According to a first aspect of the invention, there is provided a device comprising a membrane having a membrane surface area, a length, a center point with respect to the length , a width, and a center point with respect to the width. The membrane is adapted to be arranged in contact with a surface of the nasal cavity, and defines a closed volume. The membrane has the shape of the nasal cavity such that when in use, it expands and conforms to the nasal cavity. The device further comprises a first catheter extending along a distal-proximal axis, and having a first distal portion for introducing fluid into the closed volume. The first catheter has a first end portion, and the distal portion has at least one cranially placed hole having an area. The device further comprises a second catheter extending along the distal-proximal axis, wherein the second catheter has a second distal portion for removing fluid from the closed volume. The second catheter has a second end portion, and the distal portion has at least one cranially placed hole having a total area, and a distal bevel opening having an area, and a distal slit. The distal portion of the first catheter protrudes in the closed volume further than the distal portion of the second catheter, and the combined area of the holes on the first catheter is smaller than the total area of the combined area of the holes on the second catheter, and the membrane length extends along a portion of the distal-proximal axis such that the membrane surface area on a distal side of the center point is larger than the membrane surface area on a proximal side of the center point. The device further comprises means for circulating said fluid into said volume via said at least one cranially placed hole on the distal portion of the first catheter and out of said volume via said at least one cranially placed hole and distal opening on the distal portion of the second catheter.

One advantage of the holes being cranially placed on the first and second catheters is that it allows for an advantegous flow pattern in the membrane. Such flow pattern provides for a more comfortable experience for the patient and ensures that the fluid flowing in the membrane is optimally disposed. One advantage of having at least one hole, not cranially placed, on the second catheter is that it allows air to escape. One advantage of having a total area of the openings on the first catheter being smaller than the total area of the openings on the second catheter, is that it esnures that the catheter removing fluid from the membrane has a lower flow resistance than the catheter introducing fluid. This contributes to maintaining the right pressure level in the membrane, thus making it more comfortable for the patient. This advantage is further enhanced by the distal slit, and the distal bevel opening on the distal portion on the second catheter which provides an opening for removing fluid from the membrane which is allowed to be bigger than the diameter of the catheter. Another advantage of having a distal slit on the distal portion of the second catheter is that if the membrane is in touching contact with tissue in the nasal cavity such that the cranially placed holes are obstructed by the membrane, the slit still allows for the removal of fluid. This ensures that the pressure in the membrane does not get too high. This is especially usefol for patients having an abnormal nasal cavity anatomy. One advantage of having a membrane surface area on a distal side of the center point being larger than the mebrane surface area on a proximal side of the center point is that the flow pattern in the membrane when circulating the fluid becomes more optimal. This is achieved by having a larger membrane area where the fluid is introduced in the membrane and a smaller area where it is removed. This mebrane shape causes a flow pattern which reduces the risk of the circulating fluid becoming stagnant in the membrane. Thus, the pressure and circulation of the fluid in the membrane is maintained which allows for efficient continuous cooling of the brain.

According to another aspect of the invention, the center point is located at equal distance from the first and second end portions. An advantage of this is that the flow pattern in the membrane is optimal for heat exhchange between the fluid and its surroundings.

According to another aspect of the invention, the center point is located closer to the first end portion than to the second end portion. An advantage of this is that the flow pattern in the membrane is optimal for heat exhchange between the fluid and its surroundings.

According to another aspect of the invention, the membrane surface area on one side of the center point is larger than the membrane surface area on the other side of the center point such that the at least one cranially placed hole faces the larger side. An advantage of this is that the flow pattern in the membrane is optimal for heat exhchange between the fluid and its surroundings. Another advantage is that the fluid is less likely to become stagnant in the membrane.

According to another aspect of the invention, the at least one cranially placed hole on the first catheter each have an individual area of between 0.1 - 1.5 mm².

According to another aspect of the invention, the at least one cranially placed hole on the second catheter each have an individual area of between 0.1 - 1.5 mm².

According to another aspect of the invention, the number of holes on the first catheter equals or exceeds the number of holes on the second catheter.

According to another aspect of the invention, the number of holes on the second catheter equals or exceeds the number of holes on the first catheter. An advantage of this is that the flow resistance can be lower for the fluid leaving the membrane, which ensures that the pressure in the membrane does not get too high.

According to another aspect of the invention, the number of at least one cranially placed hole on the first catheter is at least 2. An advantage of this is that it ensures filling of the membrane, such that it inflates like a balloon. Another advantage of this is that fluid can enter the membrane at a satisfying rate, thus enabling for a circulation of fluid that is efficient and comfortable for the patient.

According to another aspect of the invention, the number of at least one cranially placed hole on the second catheter is at least 2. An advantage of this is that the flow resistance for fluid leaving the membrane is low. Another advantage of this is that fluid can leave the membrane at a satisfying rate, thus enabling for a circulation of fluid that is efficient and comfortable for the patient.

According to another aspect of the invention, the center point with respect to the length is located 1.5 times closer to the first end portion than to the second end portion. An advantage of this is that the flow pattern in the membrane is optimal for heat exhchange between the fluid and its surroundings. Another advantage is that the fluid is less likely to become stagnant in the membrane.

According to another aspect of the invention, the center point with respect to the length is located 2 times closer to the first end portion than to the second end portion. An advantage of this is that the flow pattern in the membrane is optimal for heat exhchange between the fluid and its surroundings. Another advantage is that the fluid is less likely to become stagnant in the membrane.

According to another aspect of the invention, the membrane surface area on the side of the center point faced by the at least one cranially placed hole is 2 times larger than the membrane surface area on the other side of the center point. An advantage of this is that the flow pattern in the membrane is optimal for heat exhchange between the fluid and its surroundings. Another advantage is that the fluid is less likely to become stagnant in the membrane.

In a non-claimed aspect which is not part of the present invention, there is provided a method for indirect temperature regulation of the brain of a human via a nasal cavity thereof, the method comprising the steps of: introducing a membrane into a nasal cavity, the membrane having a membrane surface area, a length, a center point with respect to the length, a width, and a center point with respect to the width. The membrane is adapted to be arranged in contact with a surface of the nasal cavity, and defines a closed volume. The membrane has the shape of the nasal cavity such that when in use, it expands and conforms to the nasal cavity. The device further comprises a first catheter extending along a distal-proximal axis, and having a first distal portion for introducing fluid into the closed volume. The first catheter has a first end portion, and the distal portion has at least one cranially placed hole having an area. The device further comprises a second catheter extending along the distal-proximal axis, wherein the second catheter has a second distal portion for removing fluid from the closed volume. The second catheter has a second end portion, and the distal portion has at least one cranially placed hole having an area, and a distal bevel opening having an area, and a distal slit. The distal portion of the first catheter protrudes in the closed volume further than the distal portion of the second catheter, and the combined area of the holes on the first catheter is smaller than the total area of the combined area of the holes on the second catheter, and the membrane length extends along a portion of the distal-proximal axis such that the membrane surface area on a distal side of the center point is larger than the membrane surface area on a proximal side of the center point. The device further comprises means for circulating said fluid into said volume via said plurality of cranially placed holes on the distal portion of the first catheter and out of said volume via said cranially placed holes and distal opening on the distal portion of the second catheter. The method further comprises circulating said fluid into said volume via said plurality of cranially placed holes on the distal portion of said first catheter and out of said volume via said cranially placed holes and distal opening on the distal portion of said second catheter, by aspiration. The method further comprises regulating said fluid by comparing the power of the fluid introduced in the membrane with the fluid leaving the membrane by using a flow meter.

An advantage of circulating the fluid by aspirating fluid from the second catheter is that it is a safe and easy way of controlling the pressure in the membrane. By aspirating fluid from the second catheter it also becomes possible to assess the pressure in the membrane by studying the flutter of the small portion of the membrane that is visible externally. No flutter indicates that the pressure is too high, and the medical proffesional can adjust the flow accordingly. Likewise, if the flutter is too vivid, it's an indication of the membrane not having enough pressure.

An advantage of comparing the heat flow of the fluid introduced in the membrane with the fluid leaving the membrane using a flow meter is that it gives an indication of the temperature change in the brain. When the power difference bewteen the fluid introduced in the membrane and the fluid leaving the membrane is zero, the temperature of the brain has succesfully been altered. This allows for a way of indicating the brain temperature change non-invasively. Another advantage of utilizing flow meters for sensing the rate of heat flow in the fluid leaving and being introduced in the membrane, is that it gives a fast indication of the temperature change in the brain without requiring temperature sensor feedback. Thus, when connected to the temperature regulator, this way of sensing the heat flow offers a quick feedback and enables a very fast regulation of the temperature.

According to another aspect of the invention, the method further comprises the step of sensing the temperature of the brain by using a temperature sensor adapted to be positioned adjacent to the corner of the eye. An advantage of sensing the temperature is that it in combination with the flow meter gives an improved feedback of the efficiency of the temperature regulation.

Preferred embodiments appear in the claims and in the description. It is noted that the invention relates to all possible combinations of features unless explicitly stated otherwise.

### Brief description of the drawings

The invention will by way of example be described in more detail with reference to the appended schematic drawings, which show presently preferred embodiments of the invention.
Figure 1 shows a schematic elevated view of a device for indirect temperature regulation of the brain according to the invention.
Figure 2 shows a side perspective view of a device according to the invention in a nasal cavity of a patient.
Figure 3 shows a schematic view of a system comprising the device according to the invention.
Figure 4 shows a flow-chart of a method for indirect temperature regulation of the brain according to the invention.

### Detailed description of preferred embodiments

It is contemplated that there are numerous modifications of the embodiments described herein, which are still within the scope of the invention as defined by the appended claims.

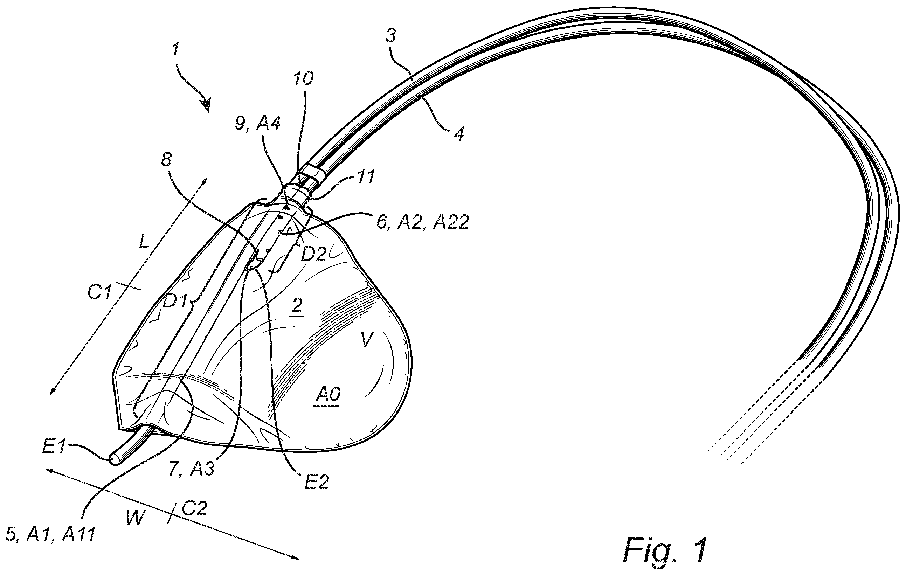

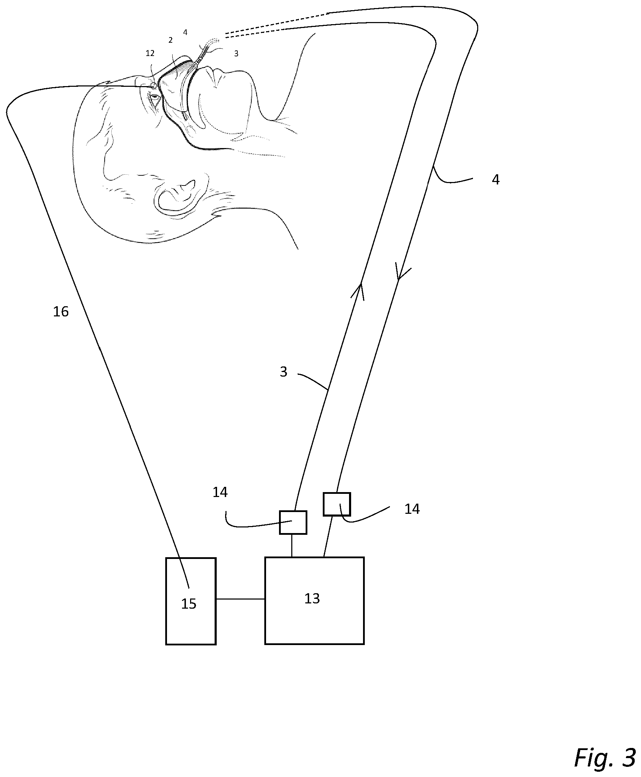

Figure 1 shows a schematic elevated view of a device 1 for indirect temperature regulation according to the invention. The device comprises a membrane 2. The membrane 2 has a membrane surface A0, and a length L and width W. The length L and width W can vary from patient to patient as the nasal cavity is individual. Preferably, the length is 100mm-200mm, and the width 50mm-200mm. As can be seen in figure 1, the membrane 2 has a center point C1 with respect to the length L, and a center point C2 with respect to the width. In figure 1, the center point C1 and C2 are both located in the middle of the length L and the width W respectively. The membrane 2 is adapted to be inserted through a patient's nostril to be in contact with a surface of the nasal cavity. When the membrane 2 is inserted, it is deflated. When in use, the membrane 2 is filled with a fluid, thus making it expand like a balloon. The membrane defines a closed volume V. The membrane 2, as can be seen in figure 1, has a shape that mimics the shape of the nasal cavity. Thus, when in use, the membrane 2 expands and conforms to the nasal cavity. The device 2 also comprises two catheters 3,4. The first catheter 3 extends along a distal-proximal axis, such that the distal portion D1 of the catheter 3 is inserted in the nasal cavity. The catheter 3 has an end portion E1, which constitutes the most distal portion of the catheter 3. The distal portion D1 has at least one hole 5 for introducing a fluid into the closed volume V. The fluid is preferably a saline solution, or any other fluid with adequate properties that are not harmful for the patient, should it leak. In figure 1, the distal portion D1 comprises three holes 5, however, there could also be one, two, four or more holes. Preferably, there are eight holes 5. The holes 5 in figure 1 are cranially placed. Cranially placed is to be interpreted as being placed such that when fluid from the catheter 3 enters the membrane 2 through the holes 5, the direction of flow is towards the cranium. The cranially placed holes 5 each have an individual area A11, and a combined area A1. The hole(s) 5 preferably have an individual area A11 of 1mm². It is however plausible that the area A11 is smaller or larger, such as 0,5mm² or 1,5mm², depending on the size of the patient's nasal cavity and thus the length of the distal portion D1, and the number of holes 5. The device 1 further comprises a second catheter 4 for removing fluid from the volume V. The first catheter 3 and second catheter 4 are in fluid communication, and preferably made out of a flexible material, such as plastic, synthetic latex, silicone or Gore-tex. It is possible that the material is coated with a substance resulting in a hydrophilic surface, or an anaesthetic agent. The first and second catheter are connected to an external pump 13, further described herein with reference to figure 3. The pump 13, catheters 3, 4 and membrane 2 form a closed system, in which the membrane 2 acts as a reservoir. The pump 13 is further connected to a temperature regulator 15 for regulating the temperature of the fluid entering the system. The temperature regulator 15 may also comprise software for automatically regulating the temperature in response to measured parameters, such as heat flow. To lower the pressure in the volume V, the flow velocity of the pump 13 is increased, which causes fluid to be aspirated and removed from the volume V through the second catheter 4. Similarly, to increase the pressure in the volume V, the flow velocity of the pump 13 is lowered. The second catheter 4 also extends along the distal-proximal axis such that the distal portion D2 of the catheter 4 is inserted in the nostril. As can be seen in figure 1, the distal portion D1 of the first catheter 3 protrudes in the closed volume V of the membrane 2 further than the distal portion D2 of the second catheter 4. In figure 1, the distal portion D2 of the second catheter 4 protrudes in the closed volume V about a third of the length L. It is however plausible that the distal portion D2 protrudes even further, or less, in the volume V. The second catheter 4 has an end portion E2, which is a distal bevel opening 7. The distal bevel opening 7 has an area A3. When the opening is a distal bevel opening, the area A3 can be increased since it is no longer limited to the diameter of the catheter 4. By increasing the area A3, the flow resistance for the fluid entering the opening 7, i.e. being removed from the volume V, is decreased. The distal portion D2 comprises at least one cranially placed hole 6 having a total combined area A2. The hole(s) 6 preferably have an individual area A22 of 1,2mm². It is however plausible that the area A22 is smaller or larger, such as 0,5mm² or 1,5mm², depending on the size of the patient's nasal cavity and thus the length of the distal portion D2, and the number of holes 6. In figure 1, two cranially placed holes 6 can be seen. It is equally plausible that there are more, or fewer, holes. Preferably, there are six holes 6, however, any number of holes 5, 6 is plausible, as long as the total area A2, A3 and A4 exceeds the total area A1. The distal portion D2 further comprises a distal slit 8. The distal slit is preferably 1mm-2mm. The fluid in the volume V is removed from the membrane 2 through the holes 6, opening 7 and slit 8 of catheter 4. To ensure that the pressure in the membrane does not get too high, i.e. painful for the patient, the total area A2 of the hole(s) 6, the bevel opening 7 and the slit 8 on the second catheter 4, exceeds the total area A1 of the hole(s) 6 on the first catheter 3. The slit 8 ensures that when fluid is removed from the volume V by aspiration through the holes and openings 6, 7, the pressure in the membrane 2 does not get too high, even if the anatomy of the nasal cavity is such that it obstructs fluid from exiting through the cranially placed holes 6. The membrane length L extends along a portion of the distal-proximal axis, and the membrane 2 has an opening 10 through which the first catheter 3 and second catheter 4 are introduced. The opening 10 has a diameter slightly smaller than the combined diameters of the first and second catheters such that the membrane 2 is kept tightly in place with no leakage. The opening 10 is further secured to the catheters by a glue joint 11. The distal portion D2 of the second catheter 4 further comprises holes 9, positioned in close proximity to the glue joint 11. These holes 9 ensure that air can leave the membrane 2 when aspirating the fluid. When the membrane 2 is inserted in a nasal cavity of a patient, the glue joint 11 and thus the holes 9 will become the highest point of the device 1. In figure 1, two holes 9 are illustrated, it could however be more or fewer holes, such as one, three, four or five. As can be further seen in figure 1, the membrane surface area A0 on the distal side of the center point C1 is larger than the membrane area A0 on the proximal side of the center point. The center point C1 is in figure 1 located at equal distance from the first end portion E1 and second end portion E2. However, C1 could also be located closer to the first end portion E1 than to the second end portion E2, or vice versa. As can be seen in figure 1, the membrane surface area A0 on one side of the center point C2 with respect to the width W is larger than the membrane surface area A0 on the other side of the center point C2, such that the cranially placed holes 5, 6 face the larger side of the membrane 2. The fluid is removed from the closed volume V through the holes 6, opening 7 and slit 8 in the second catheter 4 by aspiration. Since the device is part of a closed system, fluid is then correspondingly introduced into the volume V by the holes 5 on the first catheter 3. When the membrane surface area A0 is larger on one side of the center point C1 and C2, such that the cranially placed holes 5, 6 faces the larger side, the fluid flowing in the membrane will be less likely to become stagnant in the membrane. Having a smooth continuous flow in the membrane is crucial for achieving optimal brain cooling. Further, the device 1 might comprise a flow meter (not shown) for measuring the rate of heat flow of the fluid being introduced in the membrane 2 through the first catheter 3, and the fluid leaving the membrane 2 through the second catheter 4. If the flow meter shows that the heat flow of the fluid entering the membrane 2 is equal to the heat flow of the fluid leaving the membrane 2, this is an indication that the brain temperature has successfully been altered. The device 1 may also further comprise a temperature sensor 12 placed adjacent to the corner of the eye. This temperature sensor 12 may be connected, wirelessly or by wires 16, to the temperature regulator 15 such that the temperature of the fluid in the membrane 2 can be regulated or maintained.

Although figure 1 only discloses one single device 1, it is preferable that the patient has two devices 1, one in each nasal cavity.

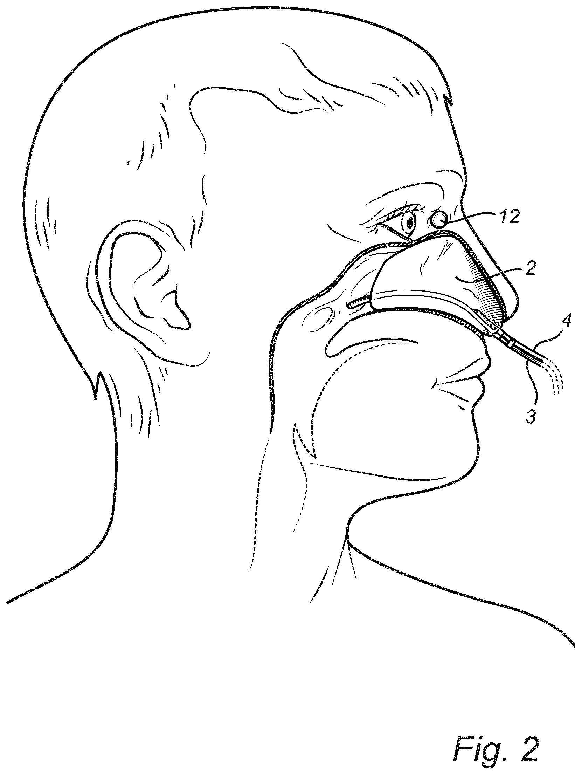

Figure 2 shows a side perspective view of the device according to the invention in a nasal cavity of a patient. As can be seen in figure 2, the membrane 2 has a shape corresponding to the anatomy of the nasal cavity of the patient. The device 1 displayed in figure 2 also comprises a temperature sensor 12 placed on the skin adjacent to the patient's eye. The sensor 12 senses the temperature of the brain, and is connected to the temperature regulator 15. By continuously measuring the temperature with the temperature sensor 12, the cooling effect of the fluid circulating in the membrane 2 can be monitored, and the temperature regulator 15 can be adjusted accordingly.

Figure 3 shows a schematic view of a system comprising the membrane 2 when it is inserted in a patient. In figure 3, the patient is lying on his back with the membrane 2 inserted into one nasal cavity. The arrows in figure 3 indicate the flow direction of the fluid being introduced in the membrane 2 through the first catheter 3, and removed through the second catheter 4. The pump 13 circulates the fluid in the system by acting as an aspirator, and sucks the fluid from the second catheter 4 rather than pumping fluid into the first catheter 3. The pump 13 is connected to a temperature regulator 15. The temperature regulator 15 comprises a heat exchanging device through which it controls the temperature of the cooling fluid entering the system. The temperature regulator 15 can be controlled manually by a medical professional, or comprise software which automatically adjusts the temperature of the cooling fluid entering the system. The temperature regulator 15 could be connected to a temperature sensor 12 placed on the skin adjacent to the patient's eye for sensing the temperature of the brain. The sensor 12 could be connected directly to the regulator 15 by wires 16 or wirelessly. By continuously measuring the temperature with the temperature sensor 12, the cooling effect of the fluid circulating in the membrane 2 can be monitored, and the temperature regulator 15 can be adjusted accordingly. The embodiment in figure 3 comprises two flow meters 14. It is to be understood that not all embodiments of the invention comprise these flow meters. The flow meters 14 measure the rate of heat flow entering the membrane 2, and leaving the membrane 2. By comparing the results an indication is given on the temperature change in the brain. When the difference between the fluid entering the membrane 2 and leaving the membrane 2 is small, this is an indication that the temperature in the brain has been altered. In figure 3, the patient is disclosed as having one device 1, it is however preferable that the patient has two devices 1 during treatment, one in each nasal cavity, for optimised cooling effect. When combining the device 1 with flow meters 14 and the temperature sensors 14, an efficient device for monitoring and adjusting the temperature of the brain can be achieved.

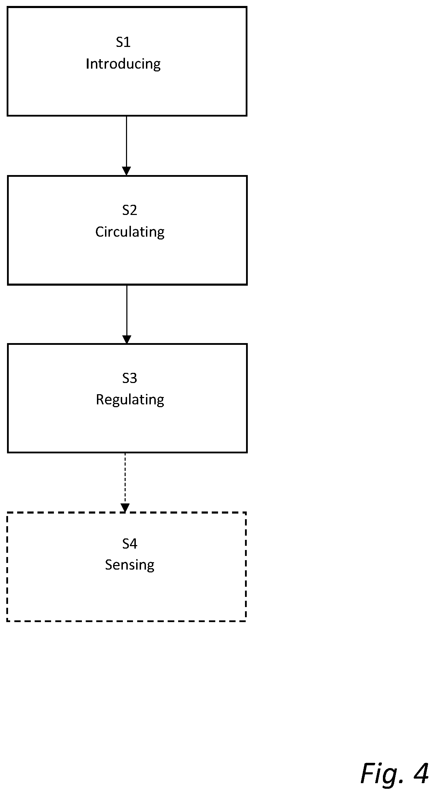

Figure 4 shows a flow-chart of a method for indirect temperature regulation of the brain according to the invention. The method comprises introducing S1 the membrane 2 into the nasal cavity of a patient. When the membrane 2 is inserted in the nasal cavity, the cooling fluid is circulated S2 into the membrane 2 through the holes 5 on the distal portion D1 of the first catheter 3, and out of the membrane 2 through the holes 6, bevel opening 7 and slit 8 on the distal portion D2 of the second catheter 4. The fluid is circulated in the system by aspiration through a pump 13. The temperature of the fluid is regulated S3 by a temperature regulator 15. The regulation S3 is controlled by a flow meter 14. The flow meter 14 compares the heat flow of the cooling fluid entering the membrane 2 with the cooling fluid leaving the membrane 2. If the difference between the fluid entering and leaving the membrane 2 is very small, the temperature of the brain has successfully been lowered. Preferably, the method also comprises sensing S4 the temperature of the brain by using a temperature sensor 12 adapted to be positioned adjacent to the corner of the eye.

For some embodiments there is provided a method for indirect temperature regulation of the brain of a human via a nasal cavity thereof, the method comprising the steps of: Introducing S1 a membrane 2 into a nasal cavity, the membrane comprising; a membrane surface area A0, a length L, a center point C1 with respect to the length L, a width W, and a center point C2 with respect to the width, said membrane 2 adapted to be arranged in contact with a surface of the nasal cavity, said membrane 2)defining a closed volume V and having the shape of the nasal cavity such that when in use, it expands and conforms to the nasal cavity, and a first catheter 3 extending along a distal-proximal axis, said first catheter 3 having a first distal portion D1 for introducing fluid into said closed volume V and a first end portion E1, said distal portion D1 having at least one cranially placed hole 5 having an area A1, and a second catheter 4 extending along the distal-proximal axis, said second catheter 4 having a second distal portion D2 for removing fluid from said closed volume V and a second end portion E2, said distal portion D2 having at least one cranially placed hole 6 having an area A2, and a distal bevel opening 7 having an area A3, and a distal slit 8, wherein the distal portion D1 protrudes in the closed volume V further than the distal portion D2, and wherein the combined area A1 is smaller than the total area of the combined area A2 and distal opening area A3, and wherein the membrane length L extends along a portion of the distal-proximal axis such that the membrane surface area A0 on a distal side of the center point C1 is larger than the membrane surface area A0 on a proximal side of the center point C1, circulating S2 said fluid into said volume V via said plurality of cranially placed holes 5 on the distal portion D1 of said first catheter 3 and out of said volume V via said cranially placed holes 6 and distal opening 7 on the distal portion D2 of said second catheter 4, by aspiration, regulating S3 said fluid by comparing the heat flow of the fluid introduced in the membrane with the fluid leaving the membrane by using a flow meter 14. According to some embodiments, the method may further comprising the step of sensing S4 the temperature of the brain by using a temperature sensor 12 adapted to be positioned adjacent to the corner of the eye.

## Claims

1. A device (1) for indirect temperature regulation of the brain of a human via a nasal cavity thereof, the device comprising:
a membrane (2) having a membrane surface area (A0), a length (L), a center point (C1) with respect to the length (L), a width (W), and a center point (C2) with respect to the width, said membrane (2) adapted to be arranged in contact with a surface of the nasal cavity, said membrane (2) defining a closed volume (V) and having the shape of the nasal cavity such that when in use, it expands and conforms to the nasal cavity,
a first catheter (3) extending along a distal-proximal axis, said first catheter (3) having a first distal portion (D1) for introducing fluid into said closed volume (V) and a first end portion (E1), said distal portion (D1) having at least one first hole (5) having a first total area (A1),
a second catheter (4) extending along the distal-proximal axis, said second catheter (4) having a second distal portion (D2) for removing fluid from said closed volume (V) and a second end portion (E2), said second distal portion (D2) having at least one second hole (6) having a second area (A2), and a distal bevel opening (7) having a third area (A3), at least one hole (9) having a fourth area (A4),
and a distal slit (8) positioned on the distal bevel opening (7), wherein the first distal portion (D1) protrudes in the closed volume (V) further than the second distal portion (D2), and wherein the first total area (A1) of the at least one first hole (5) is smaller than the total area of the second area (A2), the third area (A3), and the fourth area (A4) combined, and wherein the membrane length (L) extends along a portion of the distal-proximal axis such that the membrane surface area (A0) on a distal side of the center point (C1) is larger than the membrane surface area (A0) on a proximal side of the center point (C1), and
means for circulating said fluid into said volume (V) via said at least one first hole (5) on the first distal portion (D1) of said first catheter (3) and out of said volume (V) via said at least one second hole (6) and distal opening (7) on the second distal portion (D2) of said second catheter (4).

2. Device according to claim 1, wherein the center point (C1) is located at equal distance from the first end portion (E1) and second end portion (E2).

3. Device according to claim 1, wherein the center point (C1) is located closer to the first end portion (E1) than to the second end portion (E2).

4. Device according to any one of claims 1 - 3, wherein the membrane surface area (A0) on one side of the center point (C2) is larger than the membrane surface area (A0) on the other side of the center point (C2) such that the at least one first hole (5) faces the larger side.

5. Device according to any of the preceding claims, wherein the at least one first hole (5) each have an individual area (A11) of between 0.1 - 1 mm².

6. Device according to any of preceding claims, wherein the at least one second hole (6) each have an individual area (A22) of between 0.1 - 1mm².

7. Device according to any of the preceding claims, wherein the number of first holes (5) on the first catheter (3) equals or exceeds the number of second holes (6) on the second catheter (4).

8. Device according to any one of claims 1-6, wherein the number of second holes (6) on the second catheter (4) equals or exceeds the number of first holes (5) on the first catheter (3).

9. Device according to any of the preceding claims, wherein the number of at least one first hole (5) is at least 2.

10. Device according to any of the preceding claims, wherein the number of at least one second (6) is at least 2.

11. Device according to claim 3, wherein the center point (C1) is located 1.5 times closer to the first end portion (E1) than to the second end portion (E2).

12. Device according to claim 3, wherein the center point (C1) is located 2 times closer to the first end portion (E1) than to the second end portion (E2).

13. Device according to claim 4, wherein the membrane surface area (A0) on the side of the center point (C2) faced by the at least one first hole (5) is 2 times larger than the membrane surface area (A0) on the other side of the center point (C2).

14. Device according to claim 4, wherein the membrane surface area (A0) on the side of the center point (C2) faced by the at least one first hole (5) is 3 times larger than the membrane surface are (A0) on the other side of the center point (C2).

## Patentansprüche

1. Vorrichtung (1) zur indirekten Temperaturregelung des Gehirns eines Menschen über eine Nasenhöhle, wobei die Vorrichtung Folgendes umfasst:
Einen Membran (2) mit einem Membranoberflächenbereich (AO), einer Länge (L), einem Mittelpunkt (C1) in Bezug auf die Länge (L), einer Breite (V\/) und einem Mittelpunkt (C2) in Bezug auf die Breite, wobei die Membran (2) angepasst ist, um in Kontakt mit einer Oberfläche der Nasenhöhle angeordnet zu werden, wobei die Membran (2) ein geschlossenes Volumen (V) definiert und die Form der Nasenhöhle aufweist, so dass sie sich beim Gebrauch ausdehnt und an die Nasenhöhle anpasst,
einen ersten Katheter (3), der sich entlang einer distal-proximalen Achse erstreckt, wobei der erste Katheter (3) einen ersten distalen Abschnitt (D1) zum Einführen von Fluid in das genannte geschlossene Volumen (V) und einen ersten Endabschnitt (E1) aufweist, wobei der distale Abschnitt (D1) mindestens ein erstes Loch (5) mit einer ersten Gesamtfläche (A1) aufweist
Einen zweiten Katheter (4), der sich entlang der distal-proximalen Achse erstreckt, wobei der zweite Katheter (4) einen zweiten distalen Abschnitt (D2) zum Entfernen von Fluid aus dem geschlossenen Volumen (V) und einen zweiten Endabschnitt (E2) aufweist, wobei der zweite distaler Teil (D2) mit mindestens einem zweiten Loch (6) mit einer zweiten Fläche (A2) und einer distalen Schrägöffnung (7) mit dritten Bereich (A3), mindestens ein Loch (9) mit einem vierten Bereich (A4), und einem distalen Schlitz (8), der an der distalen Schrägöffnung (7) angeordnet ist, wobei der erste distale Abschnitt (D1) in das geschlossene Volumen (V) weiter vorsteht als der zweite distale Abschnitt (D2), und wobei die erste Gesamtfläche (A1) des mindestens einen Loch (5) kleiner ist als die Gesamtfläche der zweiten Fläche (A2), der dritten Fläche (A3) und der vierten Fläche (A4) kombiniert, und wobei sich die Membranlänge (L) entlang eines Abschnitts der distal-proximalen Achse, so dass der Membranoberflächenbereich (AO) auf einer distalen Seite des Mittelpunkts (C1) größer ist als der Membranoberflächenbereich (AO) auf einer proximalen Seite des Mittelpunkts (C1), und
Mittel zum Zirkulieren des Fluids in das Volumen (V) über das mindestens eines erstenLoches (5) in der ersten distalen Teil (D1) des ersten Katheters (3) und aus dem das Volumen (V) über das mindestens ein zweites Loch (6) und die distale Öffnung (7) an dem zweiten distalen Teil (D2) des zweiten Katheters (4)

2. Vorrichtung nach Anspruch 1, wobei der Mittelpunkt (C1) in gleichem Abstand zum ersten Endabschnitt (E1) und zum zweiten Endabschnitt (E2) angeordnet ist.

3. Vorrichtung nach Anspruch 1, wobei sich der Mittelpunkt (C1) näher am ersten Endabschnitt (E1) als zum zweiten Endabschnitt (E2) befindet.

4. Vorrichtung nach einem der Ansprüche 1 bis 3, wobei der Membranoberflächenbereich (AO) auf einer Seite des Mittelpunkts (C2) größer ist als der Membranoberflächenbereich (AO) auf der anderen Seite des Mittelpunkts (C2), so dass das mindestens ein erstes Loch (5) der größeren Seite zugewandt ist.

5. Vorrichtung nach einem der vorhergehenden Ansprüche, wobei die mindestens ein erstes Loch (5) jeweils eine individuelle Fläche (AII) zwischen 0,1 - 1 mm2aufweisen

6. Vorrichtung nach einem der vorhergehenden Ansprüche, wobei das mindestens ein zweites Loch (6) jeweils eine Einzelfläche (A22) von 0,1 - 1 mm² hat.

7. Vorrichtung nach einem der vorhergehenden Ansprüche, wobei die Anzahl der ersten Löcher (5) auf dem ersten Katheter (3) gleich oder größer ist als die Anzahl der zweiten Löcher (6) auf dem zweiten Katheter (4).

8. Vorrichtung nach einem der Ansprüche 1-6, wobei die Anzahl der zweiten Löcher (6) auf dem zweiten Katheter (4) gleich oder größer ist als die Anzahl der ersten Löcher (5) auf dem ersten Katheter (3).

9. Vorrichtung nach einem der vorhergehenden Ansprüche, wobei die Anzahl mindestens ein Loch (5) mindestens 2 beträgt.

10. Vorrichtung nach einem der vorhergehenden Ansprüche, wobei die Anzahl mindestens eine Sekunde (6) mindestens 2 ist

11. Vorrichtung nach Anspruch 3, wobei der Mittelpunkt (C1) 1,5 mal näher am ersten Endabschnitt (E1) als am zweiten Endabschnitt (E2) angeordnet ist.

12. Vorrichtung nach Anspruch 3, bei der sich der Mittelpunkt (C1) 2 mal näher an dem ersten Endabschnitt (E1) als an dem zweiten Endabschnitt (E2) befindet.

13. Vorrichtung nach Anspruch 4, wobei der Membranoberflächenbereich (AO) auf die Seite des Mittelpunkts (C2) befindet, die dem mindestens dem ersten Loch (5) zugewandt ist, ist 2 mal größer als die Membranfläche (AO) auf der anderen Seite vom Mittelpunkt (C2).

14. Vorrichtung nach Anspruch 4, wobei der Membranoberflächenbereich (AO) auf die Seite des Mittelpunkts (C2), die dem mindestens einem ersten Loch (5) zugewandt ist, ist 3 mal größer als der Membranoberflächenbereich (AO) auf der anderen Seite vom Mittelpunkt (C2).

## Revendications

1. Dispositif (1) pour la régulation indirecte de la température du cerveau d'un être humain via une cavité nasale de celui-ci, le dispositif comprenant :
une membrane (2) présentant une aire de surface de membrane (A0), une longueur (L), un point central (C1) par rapport à la longueur (L), une largeur (W), et un point central (C2) par rapport à la largeur, ladite membrane (2) étant conçue pour être agencée en contact avec une surface de la cavité nasale, ladite membrane (2) définissant un volume fermé (V) et présentant la forme de la cavité nasale de telle sorte que lors d'une utilisation, elle se déploie et épouse la cavité nasale,
un premier cathéter (3) s'étendant le long d'un axe distal-proximal, ledit premier cathéter (3) présentant une première partie distale (D1) destinée à introduire un fluide dans ledit volume fermé (V) et une première partie d'extrémité (E1), ladite partie distale (D1) présentant au moins un premier trou (5) ayant une première aire totale (A1),
un second cathéter (4) s'étendant le long de l'axe distal-proximal, ledit second cathéter (4) présentant une seconde partie distale (D2) destinée à retirer du fluide dudit volume fermé (V) et une seconde partie d'extrémité (E2), ladite seconde partie distale (D2) présentant au moins un second trou (6) ayant une deuxième aire (A2), et une ouverture biseautée distale (7) ayant une troisième aire (A3), au moins un trou (9) ayant une quatrième aire (A4), et une fente distale (8) positionnée sur l'ouverture biseautée distale (7), où la première partie distale (D1) fait davantage saillie dans le volume fermé (V) que la seconde partie distale (D2), et où la première aire totale (A1) du au moins un premier trou (5) est inférieure à l'aire totale de la deuxième aire (A2), de la troisième aire (A3) et de la quatrième aire (A4) combinées, et où la longueur de membrane (L) s'étend le long d'une partie de l'axe distal-proximal de telle sorte que l'aire de surface de membrane (A0) d'un côté distal du point central (C1) est supérieure à l'aire de surface de membrane (A0) d'un côté proximal du point central (C1), et
un moyen destiné à faire circuler ledit fluide jusque dans ledit volume (V) via ledit au moins premier trou (5) sur la première partie distale (D1) dudit premier cathéter (3) et hors dudit volume (V) via ledit au moins un second trou (6) et ladite ouverture distale (7) sur la seconde partie distale (D2) dudit second cathéter (4).

2. Dispositif selon la revendication 1, dans lequel le point central (C1) est situé à égale distance de la première partie d'extrémité (E1) et de la seconde partie d'extrémité (E2).

3. Dispositif selon la revendication 1, dans lequel le point central (C1) est situé plus près de la première partie d'extrémité (E1) que de la seconde partie d'extrémité (E2).

4. Dispositif selon l'une quelconque des revendications 1 à 3, dans lequel l'aire de surface de membrane (A0) d'un premier côté du point central (C2) est supérieure à l'aire de surface de membrane (A0) de l'autre côté du point central (C2) de telle sorte que le au moins un premier trou (5) est en face du plus grand côté.

5. Dispositif selon l'une quelconque des revendications précédentes, dans lequel chaque au moins un premier trou (5) a une aire individuelle (A11) comprise entre 0,1 et 1 mm².

6. Dispositif selon l'une quelconque des revendications précédentes, dans lequel chaque au moins un second trou (6) a une aire individuelle (A22) comprise entre 0,1 et 1 mm².

7. Dispositif selon l'une quelconque des revendications précédentes, dans lequel le nombre de premiers trous (5) sur le premier cathéter (3) est supérieur ou égal au nombre de seconds trous (6) sur le second cathéter (4).

8. Dispositif selon l'une quelconque des revendications 1 à 6, dans lequel le nombre de seconds trous (6) sur le second cathéter (4) est supérieur ou égal au nombre de premiers trous (5) sur le premier cathéter (3).

9. Dispositif selon l'une quelconque des revendications précédentes, dans lequel le nombre des au moins un premiers trous (5) est d'au moins 2.

10. Dispositif selon l'une quelconque des revendications précédentes, dans lequel le nombre des au moins un seconds trous (6) est d'au moins 2.

11. Dispositif selon la revendication 3, dans lequel le point central (C1) est situé 1,5 fois plus près de la première partie d'extrémité (E1) que de la seconde partie d'extrémité (E2).

12. Dispositif selon la revendication 3, dans lequel le point central (C1) est situé 2 fois plus près de la première partie d'extrémité (E1) que de la seconde partie d'extrémité (E2).

13. Dispositif selon la revendication 4, dans lequel l'aire de surface de membrane (A0) du côté du point central (C2) en face du au moins un premier trou (5) est 2 fois supérieure à l'aire de surface de membrane (A0) de l'autre côté du point central (C2).

14. Dispositif selon la revendication 4, dans lequel l'aire de surface de membrane (A0) du côté du point central (C2) en face du au moins un premier trou (5) est 3 fois supérieure à l'aire de surface de membrane (A0) de l'autre côté du point central (C2).
